(19) **Europäisches Patentamt European Patent Office Office européen des brevets**

(11) **EP 1 642 963 B1**

(12) **EUROPEAN PATENT SPECIFICATION**

(45) Date of publication and mention
of the grant of the patent:
**25.02.2009 Bulletin 2009/09**

(51) Int Cl.:
*C12N 1/20* (2006.01)        *A61K 35/74* (2006.01)
*A61P 9/12* (2006.01)

(21) Application number: **03817186.4**

(22) Date of filing: **25.08.2003**

(86) International application number:
**PCT/CN2003/000714**

(87) International publication number:
**WO 2004/113509 (29.12.2004 Gazette 2004/53)**

(54) **LACTOBACILLUS CASEI LC2W STRAIN AND ITS USE IN ANTIHYPERTENSIVE ASPECT**

LACTOBACILLUS CASEI-LC2W-STAMM UND SEINE VERWENDUNG GEGEN BLUTHOCHDRUCK

SOUCHE DE LACTOBACILLUS CASEI LC2W ET SON UTILISATION CONTRE L'HYPERTENSION

(84) Designated Contracting States:
**DK FR NL**

(30) Priority: **20.06.2003 CN 03129450**

(43) Date of publication of application:
**05.04.2006 Bulletin 2006/14**

(73) Proprietor: **Shanghai Bright Dairy & Food Co. Ltd.
201103 Shangai (CN)**

(72) Inventors:
• **WU, Zhengjun
Shanghai 201103 (CN)**
• **GUO, Benheng
Shanghai 201103 (CN)**
• **YE, Jin
Shanghai 201103 (CN)**

(74) Representative: **Schwahn, Hartmut
Gleiss Grosse Schrell & Partner
Patentanwälte Rechtsanwälte
Leitzstraße 45
70469 Stuttgart (DE)**

(56) References cited:
**EP-A- 0 181 170          JP-A- 05 025 055
JP-B2- 2 676 250          JP-B2- 06 047 551**

**Description**

**Field of the Invention**

The invention relates to a new kind of *lactobacillus* strain, and its use for producing and manufacturing product which has effect on anti-hypertension (and/or lowering blood pressure level).

**Background of the Invention**

According to the regulation of World Health Organization (WHO), for an adult, if his systolic blood pressure (SBP) exceeds 140 mmHg, he belongs to population of high blood pressure; if his SBP exceeds 160 mmHg, he need to be cured by medication; if his SBP ranges from 120 to 140 mmHg, he belongs to population of blood pressure on the high side. Statistical data showed that in western countries people suffering from hypertension accounted for 15% of the total population and Chinese people suffering from hypertension in 2002 were more than 130 million, which accounted for 10% of Chinese population. Among the hypertension patients, due to various reasons, only a minority of them has taken measures to treat with medicine.

Hypertension does harm to patient's heart, brain and kidney and has a close relation with sugar and lipid metabolic disturbance, so that it impairs their quality of lives distinctly. The use of antihypertensive drugs should not only lower blood pressure, but also reduce blood pressure variability (BPV), increase baroreflex sensitivity (BRS), improve heart rate variability (HRS) and improve circadian blood pressure, so as to prevent and cure hypertension complications to improve patient's quality of life.

Commonly used antihypertensive drugs are diuretic antihypertensive drugs, P -adrenergic blocking agents, calcium antagonists, drugs which affect the formation of angiotensin II, such as ACEI (angiotensin converting enzyme inhibitors) and angiotensin II receptor antagonists, etc. The range of application of some drugs is quite limited. For example, Celiprolol, a long-acting β -adrenergic blocking agents, is suitable for those hypertensives who are old or have slow heart rates since its effect on lowering heart rate is slight. Some antihypertensive drugs may have some side effects on patient during lowering hypertension, for example, the first generation calcium antagonist which is clinically widely used in China now, would not only accelerate the attenuation of conductibility and negative inotropic action so as to increase the risk of heart attack, but also lead to reflex sympathetic nerve excitation, more myocardial oxygen consumption as well as worse arrhythmia, so that it could not reduce incidence of disease and death rate effectively.

The combination of ACEI with angiotensin I converting enzyme will inhibit the formation of angiotensin II (Ang II) and slower the decomposition of bradykinin, thus resulting in vasodilation and lowering blood pressure. Lipid peroxides can damage cell membrane to induce the cell death. Captopril (a kind of ACEI, clinically used for hypertension) can help to reduce hypertension complications by significantly lowering lipid peroxides. Antihypertension treatment based on ACEI for patients in early stage can reduce the incidence and death rate of cardiovascular disease. New ACEIs such as Benazepril, Perindopril, and Lisinopril are effective not only on repairing cardiac muscle, but also on preventing the formation of reparative fibrosis. Furthermore, by distending glomerulus artery, ACEI can effectively lower the blood pressure of capillary inner glomerulus, so as to decrease the renal high perfusion pressure and reduce albumin excretion. However, 25% of patients administrated to ASEI may have durative dry coughs. Even some of them are found in the drug intolerance but to quit.

Some certain microorganisms, such as *Lactobacillus helveticus, Saccharomyces cerevisiae* and *Lactobacillus helveticus* LBK-16, when growing in milk-containing substrate, can release specific peptide fragments from lactoprotein by hydrolysis of protease excreted. These peptide fragments have activity of inhibiting angiotensin I converting enzyme (ACE) and opioid peptide, so that these microorganisms can lower blood pressure in body. For example, *Lactobacillus helveticus* and *Saccharomyces cerevisiae,* when fermentation, can produce VPP (valyl-prolyl-proline) and IPP (isoleucyl-prolyl-proline) (their $IC_{50}$s indicating ACE inhibitory action on ACE are 9 $\mu$M and 5 uM respectively). Fermented milk containing VPP and IPP has remarkable pressure-lowering effect on SHR (spontaneously hypertensive rats) and hypertensive patients. If SHR was administrated once to 5ml/kg (rat weight) of this fermented milk, their SBP can be reduced to 25 mmHg; if hypertensive patient was administrated to 95mL per day of this fermented milk for successive 8 weeks, their SBP can be reduced to $14.1\pm3.1$ mmHg. Furthermore, during growth, certain microorganisms can synthesize some substances that can also lower blood pressure of SHR. For example, if 1mg/kg (rat weight) of a polysaccharide-glycopeptide compound with a molecular weight of 180,000 Dalton, which can be separated from autolysate of *lactobacillus casei* YIT9018 cells, was administrated to SHR and RHR (renal hypertensive rats) once, the SBPs of both reduced to 10-20 mmHg after 6-12 hours. According to the recent experimental results of hypertensive animal models and a small number of hypertensive patients, products of milk fermented by specific microorganisms have mild effects on lowering blood pressure in body. In addition to relatively mild, stable and drug independent effects, these products would not change patient's weight, heart rate, erythrocyte, total cholesterol, HDL-C, LDL-C and other serum index such as total protein, glucose, urea nitrogen and creatinine, compared with that of control group. Therefore, use of certain microorganisms for reducing the risk of disease can be an easy and effective measure for daily lives of people.

**Purpose of the Invention**

Based on above topic, by use of special screening model in vitro, probiotic *lactobacillus* has been screened from *lactobacillus* in naturally fermented food (such as cheese, koumiss) as the target bacterium flora. Furthermore, effect and application of probiotic *lactobacillus* on antihypertension is studied also.

The purpose of this invention is to provide a new strain of *lactobacillus casei* LC2W, which has effect on antihypertension.

Another purpose of the invention is to disclose a use of the strain of *lactobacillus casei* LC2W according to the invention for antihypertension.

**Summary of the Invention**

The separation process of *Lactobacillus casei* LC2W according to the invention is as follows: Firstly, naturally fermented food (such as cheese or koumiss) as sample is inoculated into MRS liquid medium (selective medium for *lactobacillus,* purchased from Germany Merck Corporation) and cultured for enrichment. Secondly, the culture after enrichment is diluted with 0.5% (weight/volume ratio) of sterile peptone water to $10^5$-$10^6$ times, and inoculated into plate of MRS-salicin (purchased from Merck Corporation of Germany; glucose in MRS replaced with 1% (weight/volume ratio) of salicin) or LC agar (selective agar for *Lactobacillus casei,* its ingredients revealed by the following literature: Ravula, R.R.et al., 1998. Biotechnol. Tech, 12:819-822.), then cultured at 37°C for 72 hours in the absence of atmospheric oxygen ($H_2$: $CO_2$:$N_2$=10:5:85). Finally, when typical colony is appeared on plate, it is picked to inoculate on LC agar plate for further purification.

Single colony after purification is picked to inoculate on LC agar plate and cultured at 37°C under anaerobic condition ($H_2$:$CO_2$:$N_2$=10:5:85) for amplification. The strain acquired after amplification was preserved by cold storage.

Under aseptic atmosphere, the strain-containing fresh culture above is inoculated into 10% (weight percentage) of sterilized (at 118°C for 15 minutes) skimmed milk and cultured at 37°C for 24 hours. The product after fermentation is performed by centrifugation with speed of 4000rpm, at 4°C and for 20 minutes, and the supernatant after centrifugation is collected. Then the collected supernatant is performed by ultrafiltration with membrane which has a exclusion limit of 10,000 Dalton (VIVAFLOW 200, purchased from U.K. Vivascience Corporation). Inhibitory effect of the filtrate liquid after ultrafiltration on ACE is determined by model in vitro (Ref. to: N.Yamamoto et al., 1999. J.Dairy Sci. 82:1388-1393). Classifications of the strains whose fermented products have notable inhibitory effect on ACE in vitro are identified, wherein *Lactobacillus casei* LC2W can be obtained. Effect of thalli cell and metabolic product of *Lactobacillus casei* LC2W on lowering blood pressure of spontaneously hypertensive rats (SHRs) is also measured.

The microbiological characteristics of *lactobacillus casei* LC2W are shown in Table 1.

Table 1 Microbiological Characteristics of *Lactobacillus Casei* LC2W

| Test Item | Result | Test Item | Result |
|---|---|---|---|
| Gram staining | Positive | | |
| Cell morphology | Rod shape | Acid production of carbohydrate (continued) | |
| Gem formation | - | Mycose | + |
| Catalase | - | Xylose | - |
| Oxidase | - | Fructose | + |
| Growth in air | + | Ribose | + |
| Facultative anaerobic growth | + | Raffinose | - |
| O/F test | ferment | Lactose | + |
| Lactic acid production | + | Melizitose | - |
| Gas production of carbohydrate | | Salicin | + |
| Glucose | - | Rhamnose | - |
| Sodium gluconate | + | Esculin | + |
| Acid production of carbohydrate | | Mannose | + |

(continued)

| Test Item | Result | Test Item | Result |
|---|---|---|---|
| Glucose | + | Mannitol | + |
| Sodium gluconate | + | Maltose | + |
| Cellobiose | + | Sorbitol | + |
| Arabinose | - | Melibiose | - |
| Sucrose | + | Galactose | + |

Besides the beneficial effects of common *lactobacillus* on human's health, *Lactobacillus casei* LC2W according to the invention can also be used for manufacturing products with antihypertension function, such as dairy products that are effective for lowering high blood pressure. *Lactobacillus casei* LC2W can either exist in specific products in mixed form, or be produced in the form of pure bacterial agent such as bacterial powder, capsule and tablet.

By means of the following examples, the present invention will be described in detail. The following examples are only for explanation and the invention is not limited to the disclosed embodiment.

On 30th of October 2002, *Lactobacillus casei* LC2W according to the invention was deposited at China General Microbiological Culture Collection Center (CGMCC), which is located at Institute of Microbiology, Chinese Academy of Sciences, No. 13, Beiyitiao, Zhongguancun, Haidian District, Beijing, 100080, China; and was given the accession number of the deposit CGMCC NO. 0828.

**Description of the Invention**

**Example 1** Collection and separation of sample

Sample is homemade natural cheese or koumiss from a farm in Hulunbeier city, Inner Mongolia municipality.

Step 1 Sample collection

25 g (or 25 ml) of sample said above is added into 225 ml of 0.5% sterile peptone water, and then homogenized fully.

Step 2 Sample enrichment

5 ml of sample obtained from Step 1 is added into 100 ml of MRS broth medium, cultured overnight at 37°C. The culture obtained then is treated hereinafter.

Step 3 Separation by LC agar or MRS-salicin agar

1 ml of culture fluid obtained from Step 2 is sucked and diluted with 0.5% (weight/volume ratio) of sterile peptone water to $10^5 \sim 10^6$ times. 1 ml of cell-containing suspension after dilution is sucked into petri plate. Then LC agar or MRS-salicin agar that has cooled down at 50°C around after being melted is poured into said petri plate and then mixed fully. When the agar in petri plate coagulates entirely, the petri plate is placed at 37°C for 72 hours for anaerobic culture. During anaerobic culture, the growth of bacterial colony is observed; and after the culture, the plate is preserved at 4°C.

Step 4 Purification of strain

Colony obtained from Step 3 is picked with inoculating loop and streaked on LC agar plate for separation, then cultured at 37°C for 48-72 hours under anaerobic environment. Single colony is picked and observed under microscope, until the strain has been purified assuredly. The purified culture is preserved in 40% of sterile glycerol and on MRS slant.

Step 5 Inhibitory effect of metabolic products of the separated strain on ACE by means of model in vitro.

The separated pure strain is transferred to 10% (weight percentage) of sterile skimmed milk, and cultured at 37°C for 20-24 hours. The fermented product is performed by centrifugation with speed of 4000 rpm, at 4°C and for 20 minutes, and the supernatant after centrifugation is collected. Then the collected supernatant is performed by ultrafiltration with membrane which has a exclusion limit of 10,000 Dalton. Inhibitory effect of the ultrafiltrate liquid after ultrafiltration on ACE is determined by model in vitro (Refer to the following document: N.Yamamoto et al., 1999. J.Dairy Sci. 82: 1388-1393). Taken ultrafiltrate liquid of unfermented sterile skimmed milk as contrast group, inhibitory effect of metabolic products of every strain on ACE is calculated by the following formula. From the calculated result, those strains that have remarkable inhibitory effect on ACE can be singled out.

$$\text{Inhibitory Rate} = \frac{B-A}{B} \times 100\%$$

Wherein, A is the optical density of ACE converted products when $\lambda$ =228nm, obtained from that the fermented product sample is added into reactive system.

B is the optical density of ACE converted products when $\lambda$ =228 nm, obtained from that ultrafiltrate liquid of sterile skimmed milk is added into reactive system.

Table 2 Results of *Lactobacillus* which have inhibitory effect on ACE by means of preliminary screening

| Inhibitory Rate (%) | Number of strains | Ratio (%) | Source | |
|---|---|---|---|---|
| | | | Natural cheese | Koumiss |
| 35 | 2 | 1.6 | 2 | 0 |
| 20   34 | 5 | 4.0 | 2 | 3 |
| 10   19 | 11 | 8.8 | 5 | 6 |
| 9 | 107 | 85.6 | 42 | 65 |

Therein, inhibitory rate of the strain LC2W is the highest and up to 42%.

Step 6 Bacterial identification of the strain LC2W

As identified by Institute of Microbiology, Chinese Academy of Sciences, LC2W's microbiological characteristics are shown in Table 1.

**Effect of fermented products of *lactobacillus casei* LC2W (CGMCC NO. 0828) on lowering blood pressure of SHR**

Spontaneously hypertensive rat (SHR) is a kind of hereditable hypertensive experimental animal model and is the most general animal model used for studying on antihypertensive drugs or other blood pressure-lowering substance. By the changes of systolic pressure of rats after single or several administrations by gastric gavage, the substance's temporary and long-term effect on lowering pressure can be observed.
Product of the fermented milk of *lactobacillus casei* LC2W according to the invention after single or several administrations by gastric gavage, has remarkable effect on lowering blood pressure of SHR.

1. Experimentation

1.1 Preparation of experimental sample

*Lactobacillus casei* LC2W(CGMCC No.0828) ferments in 10% (weight percentage) of skimmed milk at 37°C for 20-24 hours, the inhibitory rate of whose fermentation supernatant against ACE can be up to 35-42%. The fermented milk obtained is firstly homogenized and secondly performed by spray drying (the temperature of intake air being 165-170°C, the temperature of outtake air being 70-72°C). The obtained spray dried powder of fermented milk then is used as the experimental sample.
10 g of spray dried powder obtained above is dissolved into 90 ml of distilled water, stirred fully, and then performed by centrifugation with the speed of 4,000 rpm at 4°C for 20 minutes. The supernatant fluid is taken to ultrafilter by ultrafiltration with membrane which has a exclusion limit of 10,000 Dalton. Inhibitory effect of the ultrafiltrate after ultrafiltration on ACE and density of the peptide are determined. The results show that the inhibitory effect of fermented milk on ACE will not be affected by treatment of spray drying (inhibitory rate of ultrafiltrate against ACE is 38 - 41%). Each gram of spray dried powder of the fermented milk contains 27 mg of active ingredient.

1.2 Experimental Animal

Spontaneously hypertensive rats(SHRs) are 18 weeks old, male and 318±33 g of body weight, provided by shanghai laboratory animal center, Chinese Academy of Sciences, the acceptance certificate of which is No. 152.

1.3 Experimental method

With SHR electronic blood pressure meter (made by Beijing Sino-Japan Friendship Hospital), the blood pressure of sober rat is measured indirectly by tail cuff manometry. The SHRs are put into a warm box at 38°C and heated for 15-20 minutes. And then their systolic pressures are measured.

The 24 spontaneously hypertensive rats are divided into 3 groups randomly, where each group is 8 rats. The SHRs of control group are administrated to distilled water; the SHRs of high dose group are administrated to 2.75 g of spray dried powder of the fermented milk for 1 kg body weight (equivalent to 75 mg of active peptide/kg body weight); and the SHRs of low dose group are administrated to 0.55 g of spray dried powder of the fermented milk for 1 kg body weight (equivalent to 15 mg of active peptide/kg body weight, since 1 g of spray dried powder of the fermented milk contains 27 mg of active ingredient). The SHRs' pressures begin to be measured a week before drug administration so as to start the experiment after the blood pressure become steady. These SHRs are administrated to drug once a day for consecutive 16 days, and their blood pressures are measured on 1st, 5th and 16th day respectively. During pre-experiment, the pressures of SHRs need to be measured before drug administration, and then at 2nd, 4th and 6th hour after drug administration by gastric gavage so as to obtain the effect of the drug, i.e. spray dried powder of the fermented milk according to the invention, on lowering blood pressure of SHR. As is shown in the result that effect of the drug on lowering blood pressure goes to peak at the 4th - 6th hour. Therefore, the time to measure blood pressure should be before drug administration and at the 5th - 6th hours after drug administration.

1.4 Data Processing

The experimental data are denoted by $\bar{x} \pm SD$, and significance testing is performed by Student t-test.

2. Experimental Result

At the first time of drug administration, the systolic pressures of SHRs of high dose group and low dose group are 192±20 mmHg and 195±7 mmHg before drug administration, then drop to 175±24 mmHg and 174±8 mmHg respectively at the 5th - 6th hours after drug administration. A noticeable decrease can be observed compared with the systolic pressure before drug administration (P<0.05 or P<0.01), as can be seen in Table 3.

Table 3 The effect of *lactobacillus casei* LC2W of low dose group and high dose group on lowering blood pressure of SHRs by gastric administration

| Experimental Groups | Blood pressure before drug administration (mmHg) | Blood pressure after drug administration (mmHg) | | |
|---|---|---|---|---|
| | | 1st day | 5th day | 16th day |
| control group | 203±12 | 199±8 | 198±8 | 201±11 |
| Low dose group | 192±20 | 175±24** | 171±18* | 181±18* |
| High dose group | 195±7 | 174±8* | 190±17 | 190±19 |

* P<0.05, ** P<0.01, compared with the systolic pressure before drug administration

During drug administration to SHRs of low dose group, *lactobacillus casei* LC2W has obvious effect on lowering blood pressure of SHR by measuring the blood pressure of SHR on 1st, 5th, and 16th day (P<0.05). While the effect of *lactobacillus casei* LC2W in high dose group on lowering blood pressure of SHR is not so perceivable on 5th and 16th day (P>0.05).

Before drug administration on 5th day (equivalent to 24 hours after drug administration of the previous day), blood pressures of SHRs of low dose group and high dose group are measured to reach 195±18 mmHg and 194±13 mmHg respectively, which have no statistically difference compared with that of control group (203±7 mmHg). In the same way, before the drug administration on 16th day (equivalent to 24 hours after drug administration of the previous day), blood pressures of SHRs of low dose group and high dose group are 200±14 mmHg and 202±16 mmHg respectively, which have no statistically difference compared with that of control group (206±9 mmHg), as can be seen in Table 4.

Table 4 The effect of *lactobacillus casei* LC2W of low dose group and high dose group on lowering blood pressure of SHRs by gastric administration

| Experimental Groups | Blood pressure before drug administration (mmHg). | | |
|---|---|---|---|
| | 1st day | 5th day | 16th day |
| Control group | 203±12 | 203±7 | 206±9 |
| Low dose group | 192±20 | 195±18 | 200±14 |
| High dose group | 195±7 | 194±13 | 202±16 |

\* P<0.05, \*\* P<0.01, compared with blood pressure before drug administration.

In addition, it can be seen from Table 3 and Table 4 that the blood pressure variance of SHRs of control group has no statistical difference.

3. Conclusion

Active ingredient of spray dried powder of the fermented milk according to the invention, i.e. LC2W, has obvious effect on lowering blood pressure of spontaneously hypertensive rats.

**Effect of thalli cell of *lactobacillus casei* LC2W (CGMCC NO. 0828) on lowering blood pressure of SHR**

1. Experimentation

1.1 Preparation of experimental sample

After *lactobacillus casei* LC2W (CGMCC No.0828) is cultured in MRS liquid medium at 37°C, thalli cells of LC2W can be obtained by centrifugation with fermented milk, then washed with sterile normal saline, and suspended again in 10% (weight percentage) of sterile remade skimmed milk, finally freeze-dried to result in lyophilized powder of thalli cells. The lyophilized powder of thalli cells is divided into two: wherein one of the lyophilized powder of thalli cells without any more treatment is used directly for drug administration to SHR by gastric lavage as LC2W-VC sample (content of live bacteria LC2W of which being $5\times10^{10}$ cfu/g) (for SHRs of LC2W-VC group); while the other of the lyophilized powder of thalli cells is dried by heat treatment at 100°C for 30 minutes, and then used for drug administration to SHR by gastric lavage as LC2W-HKC sample (content of live bacteria LC2W of which being less than $1 \times 10^4$ cfu/g) (for SHRs of LC2W-HKC group). Before drug administration, both samples are diluted with distilled water to 0.1 g/ml of suspending fluid, which are prepared freshly when need. In addition, 1.0 g of skimmed milk powder is weighed and added into 10 ml of distilled water to result in 0.1 g/ml of skimmed milk as control group, which is also prepared freshly when needed.

1.2 Experimental animal

Spontaneously hypertensive rat (SHR) is 17~18 weeks old, male and 323±23 g of body weight, provided by shanghai laboratory animal center, Chinese Academy of Sciences, the acceptance certificate of which is No. 152.

1.3 Experimental method

With SHR electronic blood pressure meter (made by Beijing Sino-Japan Friendship Hospital), the blood pressure of sober rat is measured indirectly by tail cuff manometry. The SHRs are put into a warm box at 38°C and heated for 15-20 minutes and then their systolic pressures are measured.

30 spontaneously hypertensive rats are divided into 3 groups randomly, where each group is 10 rats. The SHRs of control group are administrated to 0.4 g/kg/d of skimmed milk powder; the SHRs of LC2W-VC group and those of LC2W-HKC group both are administrated respectively to 0.4g/kg/d of theirs own samples described above. The SHRs' pressures are measured a week ago before drug administration so as to begin the experiment after the blood pressure become steady. These SHRs are administrated to drug once a day for consecutive 15 days and their blood pressures are measured on the 1st, 5th, 5th, 11th and 15th day. During pre-experiment, the SHRs' pressures need to be measured before drug administration, and then measured respectively at the 2nd, 4th and 6th hour after drug administrated by gastric lavage so as to obtain the drug's effect on SHRs. It is shown that effect of the drug on lowering blood pressure goes to peak at the 4th hour around. Therefore, blood pressure of SHRs during the following experiment should be measured before drug administration and at 4th hours after drug administration.

1.4 Data Processing

The experimental data is denoted by $\bar{x} \pm SD$, significance testing is performed by Student t-test.

2. Experimental Result

At the first time of drug administration, the systolic pressures of SHRs of LC2W-VC group and LC2W-HKC group are respectively $187 \pm 12$ mmHg and $196 \pm 14$ mmHg before drug administration; then drop to respectively $175 \pm 24$ mmHg and $174 \pm 8$ mmHg at the 4th hours after drug administration. A noticeable decrease in systolic pressure of SHR can be seen compared with that before drug administration (P<0.01), as can be shown in Table 5.

During the 15 days' administration to SHR of LC2W-VC group and LC2W-HKC group, lyophilized powder of thalli cells has obvious effect on lowering blood pressure on the 4th, 8th, 11th and 15th day (P<0.01), as can be seen in Table 5.

Table 5 The effect on blood pressure of SHRs administrated to LC2W-VC sample and LC2W-HKC sample by gastric lavage

| Experimental groups | Blood pressure before administration (mmHg) | Blood pressure after administration mmHg | | | | |
|---|---|---|---|---|---|---|
| | | 1st day | 4th day | 8th day | 11th day | 15th day |
| Control group | $200 \pm 18$ | $195 \pm 16$ | $193 \pm 20$ | $188 \pm 14$ | $189 \pm 19$ | $194 \pm 16$ |
| LC2W-VC group | $187 \pm 12$ | $175 \pm 12^{**}$ | $171 \pm 12^{**}$ | $174 \pm 9^{**}$ | $173 \pm 11^{**}$ | $178 \pm 9^{**}$ |
| LC2W-HKC group | $196 \pm 14$ | $175 \pm 8^{**}$ | $178 \pm 10^{**}$ | $179 \pm 6^{**}$ | $171 \pm 8^{**}$ | $171 \pm 5^{**}$ |

* P<0.05, ** P<0.01, compared with blood pressure of SHR of the control group.

Before drug administration on the 4th day (equivalent to 24 hours after drug administration of the previous day), blood pressures of SHRs of LC2W-VC and LC2W-HKC group as measured are respectively $188 \pm 11$ mmHg and $192 \pm 9$ mmHg, which have no statistical difference with that of control group ($195 \pm 24$ mmHg). In the same way, before drug administration on the 8th, 11th and 15th day (equivalent to 24 hours after drug administration of the previous day), blood pressures of SHRs between experimental groups and control group have no statistical difference (P>0.05), as can be seen in Table 6.

Table 6 The effect on blood pressure of SHRs administrated to LC2W-VC sample and LC2W-HKC sample by gastric lavage

| Experimental groups | Blood pressures before administration (mmHg) | | | | |
|---|---|---|---|---|---|
| | 1st day | 4th day | 8th day | 11th day | 15th day |
| Control group | $200 \pm 18$ | $195 \pm 24$ | $190 \pm 16$ | $190 \pm 16$ | $195 \pm 17$ |
| LC2W-VC group | $187 \pm 12$ | $188 \pm 11$ | $189 \pm 11$ | $192 \pm 12$ | $192 \pm 10$ |
| LC2W-HCK group | $196 \pm 14$ | $192 \pm 9$ | $191 \pm 7$ | $192 \pm 6$ | $192 \pm 8$ |

* P<0.05, ** P<0.01, compared with blood pressure of SHRs of control group before drug administration at the same day.

3. Conclusion

Thalli cell and active ingredient of *lactobacillus casei* LC2W have obvious effect on lowering blood pressure of spontaneously hypertensive rats.

**Use of *lactobacillus casei* LC2W for producing *lactobacillus* beverage or live bacteria milk beverage which has function of lowering blood pressure**

1. Raw milk (such as skimmed milk, fresh milk, remade milk) is treated twice with high-strength heat at 95°C for 20 minutes or at 140°C for 2 seconds. Then *lactobacillus casei* LC2W (CGMCC NO.0828) according to the invention is added into said milk so that the content of LC2W in milk reaches $10^9$ cfu/ml. Finally sweet live bacterial milk beverage can be obtainable by cold preservation of the milk at 5°C.

2. Raw milk (such as skimmed milk, fresh milk, remade milk) is treated twice with high-strength heat at 95°C for 20 minutes or at 140°C for 2 seconds, and then cooled to 37°C. *Lactobacillus casei* LC2W(CGMCC NO.0828) is inoculated into the cool milk at inoculum size of 5% (volume percentage), then cultured at 37°C till the final acidity reaches 0.6-0.7% (lactic acid). Finally fermented milk beverage can be obtainable by cold preservation of the milk at 5°C.

**Claims**

1. A kind of *Lactobacillus casei* LC2W, the accession number of the deposit of which is CGMCC NO.0828.

2. Use of *Lactobacillus casei* LC2W (CGMCC NO. 0828) as claimed in Claim 1 for the preparation of a medicament for antihypertension.

**Patentansprüche**

1. *Lactobacillus casei* LC2W-Stamm mit der Hinterlegungsnummer CGMCC Nr. 0828.

2. Verwendung von *Lactobacillus casei* LC2W (CGMCC Nr. 0828) gemäß Anspruch 1 zur Herstellung eines Medikaments gegen Bluthochdruck.

**Revendications**

1. Souche de *Lactobacillus casei* LC2W avec le numéro d'ordre du dépôt CGMCC n°0828.

2. Utilisation de *Lactobacillus casei* LC2W (CGMCC n° 0828) selon la revendication 1 pour la préparation d'un médicament contre l'hypertension.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RAVULA,R.R. et al.** *Biotechnol. Tech,* 1998, vol. 12, 819-822

- **N.YAMAMOTO et al.** *J.Dairy Sci.,* 1999, vol. 82, 1388-1393